(19) Europäisches Patentamt
European Patent Office
Office européen des brevets

(11) Veröffentlichungsnummer: **0 230 244**
**A2**

(12) # EUROPÄISCHE PATENTANMELDUNG

(21) Anmeldenummer: **87100289.5**

(51) Int. Cl.⁴: **C07D 239/48** , A01N 43/54

(22) Anmeldetag: **12.01.87**

(30) Priorität: **22.01.86 DE 3601800**

(43) Veröffentlichungstag der Anmeldung:
**29.07.87 Patentblatt 87/31**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Schwamborn, Michael, Dr.**
**von Lohe-Strasse 9**
**D-5000 Köln 80(DE)**
Erfinder: **Klauke, Erich, Dr.**
**Eichendorffweg 6**
**D-5068 Odenthal(DE)**
Erfinder: **Schmidt, Robert R., Dr.**
**Im Waldwinkel 110**
**D-5060 Bergisch-Gladbach 2(DE)**
Erfinder: **Santel, Hans-Joachim, Dr.**
**Gruenstrasse 9a**
**D-5090 Leverkusen(DE)**
Erfinder: **Strang, Robert Harry, Dr.**
**Unterdorfstrasse 6A**
**D-4000 Düsseldorf 31(DE)**

(54) **Neue 2,4-Diamino-6-halogenalkylpyrimidine.**

(57) Die vorliegende Erfindung betrifft neue 2,4-Diamino-6-halogenalkylpyrimidine der allgemeinen Formel (I),

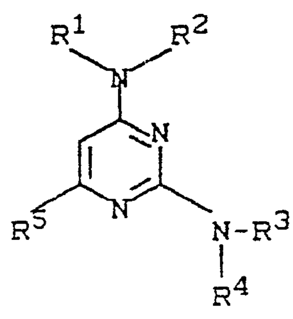

in welcher
$R^1$ und $R^3$ unabhängig vonienander für Wasserstoff oder Alkyl stehen,
$R^2$ und $R^4$ unabhängig voneinander für Wasserstoff, für gegebenenfalls durch Halogen, Cyano, Alkoxy, Alkylthio oder durch Cyclopropyl substituiertes Alkyl, für Cycloalkyl, für gegebenenfalls durch Halogen substituiertes Alkenyl oder für Alkinyl stehen und
$R^5$ für durch Halogen substituiertes Alkyl steht, mit der Maßgabe, daß $R^1$, $R^2$, $R^3$ und $R^4$ nicht gleichzeitig für Wasserstoff stehen, wenn $R^5$ für Trichlormethyl steht,
Verfahren zu ihrer Herstellung und ihre Verwendung als Herbizide.

EP 0 230 244 A2

Xerox Copy Centre

## Neue 2,4-Diamino-6-halogenalkylpyrimidine

Die vorliegende Erfindung betrifft neue 2,4-Diamino-6-halogenalkylpyrimidine, Verfahren und neue Zwischenprodukte zu ihrer Herstellung sowie ihre Verwendung als Pflanzenschutzmittel, insbesondere als Herbizide.

Es ist bereits bekannt, daß bestimmte 2,4-Diaminopyrimidine, z.B. 2,4-Diamino-6-chlor-5-methylthipyrimidin oder 2-(3-Methyoxypropylamino)-4-cyclopropylamino-6-chlorpyrimidin, als Herbizide eingesetzt werden könne (vgl. EP-A 0 000 681, DE-OS 2 006 145, DE-OS 2 630 140). Ihre Wirkung bei niedrigen Aufwandmengen ist jedoch bei verschiedenen Schadpflanzen nicht befriedigend.

Es wurden nun neue 2,4-Diamino-6-halogenalkyl-pyrimidine der allgemeinen Formel (I),

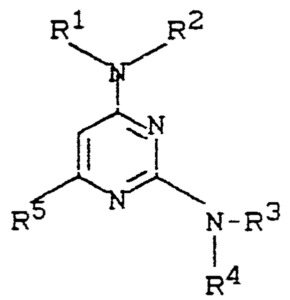

in welcher
$R^1$ und $R^3$ unabhängig voneinander für Wasserstoff oder Alkyl stehen,
$R^2$ und $R^4$ unabhängig voneinander für Wasserstoff, für gegebenenfalls durch Halogen, Cyano, Alkoxy, Alkylthio oder durch Cyclopropyl substituiertes Alkyl, für Cycloalkyl, für gegebenenfalls durch Halogen substituiertes Alkenyl oder für Alkinyl stehen,
$R^5$ für durch Halogen substituiertes Alkyl steht, mit der Maßgabe, daß $R^1$, $R^2$, $R^3$ und $R^4$ nicht gleichzeitig für Wasserstoff stehen, wenn $R^5$ für Trichlormethyl steht, und
mit der Maßgabe, daß $R^1$ und $R^2$ nicht gleichzeitig für Wasserstoff stehen, wenn $R^4$ für Alkyl steht, welches durch Alkoxy oder Alkylthio substituiert ist, aufgefunden.

Weiterhin wurde gefunden, daß man die 2,4-Diamino-6-halogenalkyl-pyrimidinderivate der allgemeinen Formel (I) erhält, wenn man Pyrimidine der allgemeinen Formel (II),

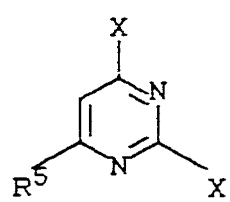

(II)

in welcher
$R^5$ die oben angegebene Bedeutung hat und
X für Chlor oder Fluor steht,
zunächst mit einem Amin der Formel (III)

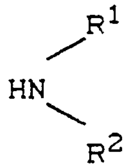

(III)

worin
$R^1$ und $R^2$ die oben angegebene Bedeutung haben,
gegebenenfalls in Gegenwart eines säurebindenden Mittels und gegebenenfalls in Gegenwart eines Verdünnungsmittels umsetzt zu einem Gemisch der isomeren Pyrimidinderivate der allgemeinen Formel - (IVa) und (IVb) (1. Stufe)

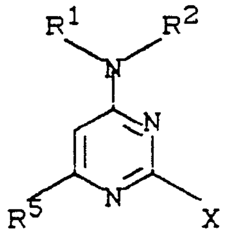

(IVa)

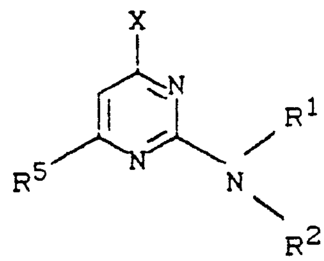

(IVb)

worin
$R^1$, $R^2$, $R^5$ und X die oben angegebene Bedeutung haben,
und anschließend gegebenenfalls nach Abtrennung der strukturisomeren Pyrimidinderivate der Formel - (IVb) in einem weiteren Reaktionsschritt mit Aminen der Formel (V),

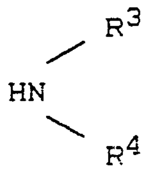

(V)

worin
$R^3$ und $R^4$ die oben angegebene Bedeutung haben,
in Gegenwart eines säurebindenden Mittels und gegebenenfalls in Gegenwart eines Verdünnungsmittels zu Pyrimidinderivaten der Formel (I) umsetzt (2. Stufe).

Außerdem wurde gefunden, daß die neuen 2,4-Diamino-6-halogenalkylpyrimidinderivate der allgemeinen Formel (I) starke herbizide Eigenschaften aufweisen.

Die erfindungsgemäßen neuen 2,4-Diamino-6-halogenalkyl-pyrimidinderivate der Formel (I) zeichnen sich gegenüber den vorbekannten Pyrimidinen strukturell insbesondere dadurch aus, daß die 6-Stellung durch eine Halogenalkylgruppe substituiert ist sowie die 5-Stellung ein Wasserstoffatom aufweist.

Überraschenderweise sind die erfindungsgemäßen Wirkstoffe der Formel (I), bei besserer Kulturpflanzenverträglichkeit, deutlich wirksamer als die vorbekannten Pyrimidinderivate, wie z.B. 2,4-Diamino-6-chlor-5-methylthiopyrimidin (bekannt aus EP-A-0 000 681) oder 2-(3-Methoxypropylamino)-4-cyclopropylamino-6-chlorpyrimidin (bekannt aus DE-OS 2 630 140).

Von den erfindungsgemäßen 2,4-Diamino-6-halogenalkylpyrimidinderivaten der Formel (I) sind bevorzugt diejenigen, in denen
$R^1$ und $R^3$ unabhängig voneinander für Wasserstoff oder für Alkyl mit 1-6 C-Atomen stehen,
$R^2$ und $R^4$ unabhängig voneinander für Wasserstoff, für gegebenenfalls durch Fluor, Chlor, Brom, Iod, Cyano

oder Cyclopropyl substituiertes Alkyl mit 1-8 C-Atomen, für Alkoxyalkyl oder Alkylthioalkyl mit 1-6 C-Atomen je Alkylthio-bzw. Alkoxyteil und 2-8 C-Atomen je Alkylteil, für Cycloalkyl mit 3-5 C-Atomen, für gegebenenfalls durch Halogen substituiertes Alkenyl mit 3-6 C-Atomen für Alkinyl mit 3-6 C-Atomen, stehen,

$R^5$ für Halogenmethyl steht, mit der Maßgabe, daß $R^1$, $R^2$, $R^3$ und $R^4$ nicht gleichzeitig für Wasserstoff stehen, wenn $R^5$ für Trichlormethyl steht,

mit der Maßgabe, daß $R^1$ und $R^2$ nicht gleichzeitig für Wasserstoff stehen, wenn $R^4$ für Alkoxyalkyl oder Alkylthioalkyl mit 1-6 C-Atomen je Alkylthio-bzw. Alkoxy und 2-8 C-Atomen je Alkylteil steht.

Aus dieser Stoffgruppe sind solche Verbindungen der Formel (I) besonders bevorzugt, in denen

$R^1$ und $R^3$ unabhängig voneinander für Wasserstoff oder für Alkyl mit 1-4 C-Atomen stehen,

$R^2$ und $R^4$ unabhängig voneinander für Wasserstoff, für gegebenenfalls durch Fluor, Chlor, Cyano oder Cyclopropyl substituiertes Alkyl mit 1-6 C-Atomen, für Alkoxyalkyl oder Alkylthioalkyl mit 1-4 C-Atomen je Alkylthio-bzw. Alkoxyteil und 2-6 C-Atomen je Alkylteil, für Cyclopropyl oder Cyclobutyl, für gegebenenfalls durch Chlor substituiertes Alkenyl mit 3-5 C-Atomen oder für Alkinyl mit 3-5 C-Atomen stehen, und

$R^5$ für durch Chlor und/oder Fluor substituiertes Methyl steht,

mit der Maßgabe, daß $R^1$, $R^2$, $R^3$ und $R^4$ nicht gleichzeitig für Wasserstoff stehen, wenn $R^5$ für Trichlormethyl steht,

mit der Maßgabe, daß $R^1$ und $R^2$ nicht gleichzeitig für Wasserstoff stehen, wenn $R^4$ für Alkoxyalkyl oder Alkylthioalkyl mit 1-4 C-Atomen je Alkylthio-bzw. Alkoxyteil und 2-6 C-Atomen je Alkylteil steht.

Ganz besonders bevorzugt sind Pyrimidinderivate der Formel (I), in denen $R^1$ und $R^3$ unabhängig voneinander für Wasserstoff, Methyl oder Ethyl stehen und $R^2$, $R^4$ und $R^5$ die als besonders bevorzugt genannten Bedeutungen haben.

Verwendet man beispielsweise 2,4-Difluor-6-trifluormethylpyrimidin und Cyclopropylamin als Ausgangsstoffe und setzt das hierbei gebildete 4-Cyclopropylamino-2-fluor-6-trifluormethylpyrimidin mit 3-Methoxypropylamin um, so kann der Reaktionsablauf zusammenfassend durch das folgende Formelschema wiedergegeben werden:

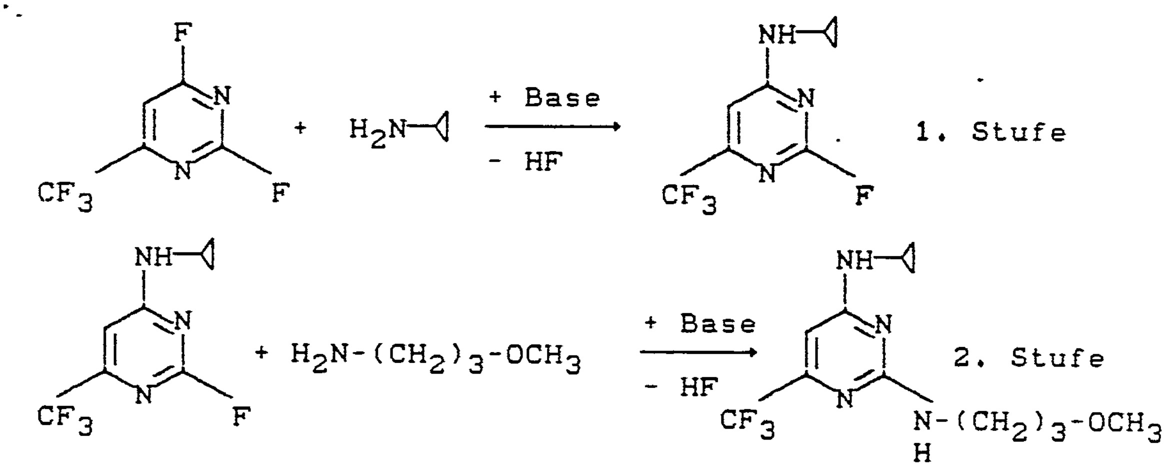


Die als Ausgangsstoffe verwendeten Pyrimidine sind durch die Formel (II) algemein definiert. In dieser Formel steht $R^5$ vorzugsweise bzw. besonders bevorzugt für diejenigen Reste, die schon bei der Beschreibung der erfindungsgemäßen Stoffe der Formel (I) als bevorzugt bzw. besonders bevorzugt für diesen Substituenten genannt wurden und X steht für Chlor oder Fluor. Die Pyrimidine der Formel (II) sind zum Teil bekannt bzw. können nach bekannten Verfahren hergestellt werden (vgl. Herstellungsbeispiele der Ausgangsstoffe).

Als Pyrimidinderivate der Formel (II) können insbesondere die folgenden Verbindungen eingesetzt werden:

2,4-Dichlor-6-trifluormethylpyrimidin,
2,4-Dichlor-6-difluorchlormethylpyrimidin,
2,4-Dichlor-6-dichlorfluormethylpyrimidin,
2,4-Dichlor-6-trichlormethylpyrimidin,

2,4-Difluor-6-trifluormethylpyrimidin,
2,4-Difluor-6-difluorchlormethylpyrimidin,
2,4-Difluor-6-dichlorfluormethylpyrimidin,
2,4-Difluor-6-trichlormethylpyrimidin.

Die weiterhin als Ausgangsstoffe verwendeten Amine sind durch die Formel (III) und (V) allgemein definiert. In diesen Formeln stehen $R^1$, $R^2$, $R^3$ und $R^4$ vorzugsweise bzw. besonders bevorzugt für diejenigen Reste, die schon bei der Beschreibung der erfindungsgemäßen Stoffe der Formel (I) als bevorzugt bzw. besonders bevorzugt für diese Substituenten genannt werden. Die Amine der Formel (III) und (IV) sind bekannt oder lassen sich nach bekannten Verfahren in analoger Weise wie die bekannten Verbindungen herstellen (vgl. z.B. Hoeben-Weyl, Methoden der organischen Chemie, Band XI/1, S. 548, S. 561 ff., 4. Auflage 1957; US-P 2 764 615).

Im einzelnen können zur Herstellung der Pyrimidinderivate der allgemeinen Formel (I) insbesondere die folgenden Amine der Formeln (III) bzw. (V) eingesetzt werden: Methylamin, Ethylamin, n-Propylamin, i-Propylamin, n-Butylamin, s-Butylamin, t-Butylamin, Neopentylamin, Dimethylamin, Diethylamin, Di-n-propylamin, Di-i-propylamin, 3-Methoxypropylamin, 3-Ethoxypropylamin, 3-n-Propoxy-propylamin, 3-i-Propoxypropylamin, 3-n-Butoxypropylamin, 3-s-Butoxypropylamin, 3-t-Butoxypropylamin, 1-Methyl-3-methoxy-propylamin, 1-Methyl-3-ethoxypropylamin, 1-Methyl-3-n-propoxypropylamin, 1-Methyl-3-i-propoxypropylamin, 1-Methyl-3-t-butoxypropylamin, 2-Methoxyethylamin, 2-Ethoxyethylamin, 2-n-Propoxyethylamin, 2-i-Propoxyethylamin, 2-n-Butoxyethylamin, 2-s-Butoxyethylamin, 2-t-Butoxyethylamin, 1-Methyl-2-methoxyethylamin, 1-Methyl-2-ethoxyethylamin, N-Methyl-N-3-methoxypropylamin, 3-Methylthiopropylamin, Allylamin, 1-Methylallylamin, 1,1-Dimethylallylamin, Proparglyamin, 1-Methylpropargylamin, Cyclopropylamin, Cyclobutylamin.

Als Verdünnungsmittel für das erfindungsgemäße Verfahren kommen organische Lösungsmittel sowie Wasser in Frage. Bevorzugte organische Lösungsmittel sind Kohlenwasserstoffe wie Toluol, aliphatische Ketone wie Aceton, Methylethylketon und Diethylketon, cycloaliphatische Ether wie Tetrahydrofuran oder Dioxan. Auch Gemische verschiedener organischer Lösungsmittel und Gemische von mit Wasser mischbaren organischen Lösungsmitteln mit Wasser sind als Verdünnungsmittel geeignet.

Das erfindungsgemäß Verfahren wird gegebenenfalls unter Verwendung von Säurebindemitteln durchgeführt. Als solche sind Erdalkali-und Alkalimetallhydroxide, wie Calcium-, Natrium-oder Kaliumhydroxid, ferner Ammoniak sowie tertiäre aliphatische Amine wie z.B. Triethylamin, aber auch im Überschuß eingesetztes Amin-Ausgangsprodukt (III) bzw. (V) besonders geeignet.

Die Reaktionstemperaturen können bei den erfindungsgemäßen Verfahren in einem größeren Bereich variiert werden. Die erste Verfahrensstufe wird im allgemeinen bei Temperaturen von -80°C bis +150°C, vorzugsweise von -80°C bis +20°C durchgeführt. Die zweite Verfahrensstufe wird im allgemeinen bei Temperaturen von -80°C bis 250°C, vorzugsweise von -20°C bis +150°C durchgeführt.

Die Umsetzung wird im Druckbereich von 1 bis etwa 10 bar durchgeführt.

Bei der Durchführung des Erfindungsgemäßen Verfahrens setzt man auf 1 Mol Pyrimidin der Formel -(II) in der ersten Stufe im allgemeinen 1 bis 2 Mol, vorzugsweise 1 bis 1,1 Mol Amin der Formel (III) und 1 bis 2 Mol, vorzugsweise 1 bis 1,2 Mol Säurebinder ein, wobei als Säurebinder das Amin der Formel (III) verwendet werden kann. Besonders bevorzugt wird unter Einsatz stöchiometrischer Molverhältnisse gearbeitet. Für die zweite Verfahrensstufe gilt Entsprechendes.

Gegebenenfalls in der ersten Stufe des Verfahrens entstandene strukturisomere Pyrimidinnebenprodukte der Formel (IVb) können in einfacher Weise nach bekannten Methoden abgetrennt werden, insbesondere durch Umkristallisation, Chromatographie oder Wasserdampfdestillation (vgl. z.B. DE-OS 2 006 145, DE-OS 2 630 140, EP 0 114 575), so daß sich die Pyrimidinderivate der Formel (IVa) in ausreichend reiner Form isolieren lassen.

Die erfindungsgemäßen Wirkstoffe können als Defoliants, Desiccants, Krautabtötungsmittl und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Spenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Anapas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Die erfindungsgemäßen Wirkstoffe können -insbesondere im Nachauflaufverfahren -zur selektiven Unkrautbekämpfung in monokotylen Kulturen, z.B. Mais, Reis, Gerste und Weizen, und dikotylen Kulturen, z.B. in Baumwolle, eingesetzt werden. Im Nachauflaufverfahren lassen sich die erfindungsgemäßen Wirkstoffe insbesondere gegen dikotyle Unkräuter einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspension-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, so also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethylharnstoff zur Unkrautbekämpfung in Getreide; 4Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit Harnstoffen wie N-Benzthiazolyl-N-methyl-N'-methylharnstoff; N,N-Dimethyl-N'-(3-chlor-4-methylphenyl)-harnstoff; N,N-Dimethyl-N'-(4-isopropylphenyl)-harnstoff; N,N-Dimethyl-N'-(3-trifluormethylphenyl)-harnstoff; Triazinen wie 2-Chlor-N{{(4-methoxy-6-methyl-1,3,5-triazin-2-yl)-amino}-carbonyl}-benzolsulfonamid; 2-Chlor-4-ethylamino-6-isopropylamino-1,3,5-triazin; 2-Chlor-4-ethylamino-6-(3-cyanopropylamino)-1,3,5-triazin; 2-Chlor-4,6-bis-(ethylamino)-1,3,5-triazin; 4-Ethylamino-2-t-butylamino-6-methylthio-s-triazin; Triazinonen wie 4-Aminc-6-tert.-butyl-3-methylthio-4,5-dihydro-1,2,4-triazin-5-on; 3-Methyl-4-amino-6-phenyl-1,2,4-triazin-5-(4H)-on; Triazindionen; Nitroanilinen wie N-(3,4-Dichlorphenyl)-2-propionamid, 2,6-Dinitro-4-trifluormethyl-N,N-dipropylanilin; N-(1-Ethylpropyl)-3,4-dimethyl-2,6-dinitroanilin; Oxyessigsäureamiden wie N-Methyl-2-(1,3-benzthiazol-2-yloxy)-acetanilid; Chloracetaniliden wie Chloressigsäure-N-(methoxymethyl)-2,6-diethylanilid, 2-Ethyl-6-methyl-N-(1-methyl-2-methoxyethyl)-chloracetanilid, α-Chlor-2',6'-diethyl-N-(2-propoxyethyl-acetanilid; Thiolcarbamaten wie N,N-Diisopropyl-S-(2,3,3-trichlorallyl)-thiolcarbamat, Benzylether; Phenoxyalkancarbonsäuren wie 2,4-Dichlorphenoxyessigsäure; 2,4-Dichlorphenoxypropionsäure, (2-Methyl-4-chlorphenoxy)-essigsäure, (4-Chlor-2-methylphenoxy)-propionsäure); Aryloxy-oder Heteroaryloxyphenoxy-alkancarbonsäuren wie 2-{4-(2,4-Dichlorphenoxy)-phenoxy}-propionsäuremethylester, 2-{4-[[3-Chlor-5-(trifluormethyl)-2-pyridinyl]-oxy]-phenoxy}-propansäure bzw. -propansäureethylester, 2-[4-(3,5-Dichlorpyrid-2-yl-oxy)-phenoxy]-propionsäure-trimethylsilylmethylester; Cyclohexandionen wie Methyl-6,6-dimethyl-2,4-dioxo-3-[1-(2-propenyloxyamino)-butyliden]-cyclohexancarbonsäure, 2-[1-(Ethoxyaminobutyliden]-5-(2-ethylthiopropyl)-1,3-cyclohexandion; Benzonitrilen wie 3,5-Diiod-4-hydroxybenzonitril; 3,5-Dibrom-4-hydroxybenzonitril; 3-Isopropyl-2,1,3-benzothiadiazin-4-on-2,2-dioxid; 6-Chlor-3-phenyl-pyridazin-4-yl-S-octyl-thiocarbonat; Pyridyloxyessigsäuren, Pyridonen wie 1-Methyl-3-phenyl-5-[3-(trifluormethyl)-phenyl]-4(1H)-pyridinon, Pyrazolen wie [4-(2,4-Dichlorbenzoyl)-1,3-dimethylpyrazol-5-yl]-4-methylphenylsulfonat kommen in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die Erfindungsgemäßen Wirkstoffe können sowohl vor als auch nach dem Auflaufen der Pflanzen appliziert werden.

Sie können auch vor der Saat in den Boden eingerarbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1

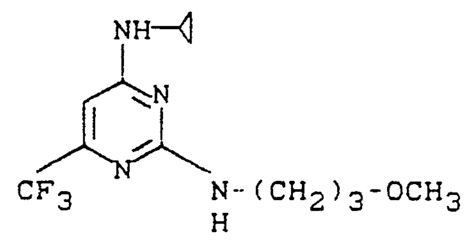

4-Cyclopropylamino-2-(3-methoxypropylamino)-6-trifluormethylpyrimidin

a) 4-Cyclopropylamino-2-fluor-6-trifluormethylpyrimidin (1. Stufe)

Zu 9,2 g (0,05 Mol) 2,4-Difluor-6-trifluormethylpyrimidin in 200 ml Tetrahydrofuran werden bei -78°C 2,9 g (0,05 Mol) Cyclopropylamin und 5 g (0,05 Mol) Triethylamin zugetropft. Das Reaktionsgemisch wird langsam auf -20°C erwärmt, 2 Stunden bei dieser Temperatur gerührt und anschließend 30 Minuten bei -10°C nachgerührt. Nach Verrühren mit 1 l Eiswasser und Extraktion mit Dichlormethan werden die vereinigten organischen Phasen getrocknet und eingeengt. Man erhält nach Zugabe von 50 ml Pentan, Absaugen und Trocknen 7,5 g (67,8 % der Theorie) des gewünschten Pyrimidin. Fp: 79-80°C.

b) 4-Cyclopropylamino-2-(3-methoxypropylamino)-6-trifluormethylpyrimidin (2. Stufe)

Zu 7 g (0,0031 Mol) 4-Cyclopropylamino-2-fluor-6-trifluormethylpyrimidin gelöst in 100 ml Tetrahydrofuran tropft man bei Raumtemperatur 6,1 g (0,068 Mol) 3-Methoxypropylamin und rührt 6 Stunden bei Raumtemperatur. Nach Zugabe von 2 l Eiswasser, Extraktion mit Dichlormethan, Trocknen der organischen Phase und Einengen erhält man 8.8 g (95.4 % der Theorie) des gewünschten Pyrimidins. n : 1.5040.

Auf analogem Wege können die in der nachfolgenden Tabelle 1 genannten Pyrimidinderivate der allgemeinen Formel (I)

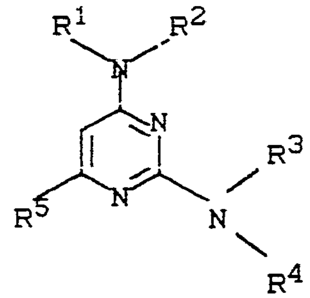

hergestellt werden:

Tabelle 1

| Beispiel-Nr. | $R^1$ | $R^2$ | $R^3$ | $R^4$ | $R^5$ | Phys. Konst. |
|---|---|---|---|---|---|---|
| 2 | H | 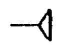 | H | $-C(CH_3)_2-CH_2-OCH_3$ | $-CF_3$ | Fp.: 74° C |
| 3 | H | $-C_3H_7-n$ | H | $-(CH_2)_3-OCH_3$ | $-CF_3$ | $n_D^{20}$ : 1,4990 |
| 4 | H | $-C_4H_9-t$ | H | $-(CH_2)_3-OCH_3$ | $-CF_3$ | Fp.: 93° C |
| 5 | H | $-C_2H_5$ | H | $-C_3H_7-i$ | $-CF_3$ | $n_D^{20}$ : 1,4989 |
| 6 | H | $-C_2H_5$ | H | $-(CH_2)_3-OCH_3$ | $-CF_3$ | $n_D^{20}$ : 1,5024 |
| 7 | H | $-C_3H_7-i$ | H | $-C_2H_5$ | $-CF_3$ | Fp.: 57° C |
| 8 | H | $-C_3H_7-i$ | H | $-(CH_2)_3-OCH_3$ | $-CF_3$ | Fp.: 67° C |
| 9 | H | 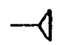 | H | $-(CH_2)_3-OCH_3$ | $-CCl_2F$ | $n_D^{20}$ : 1,5555 |
| 10 | H | 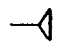 | H | $-(CH_2)_3-OCH_3$ | $-CClF_2$ | $n_D^{20}$ : 1,5232 |
| 11 | H | $-C_3H_7-i$ | H | $-(CH_2)_3-OCH_3$ | $-CClF_2$ | $n_D^{20}$ : 1,5170 |
| 12 | H | $-CH_3$ | H | $-(CH_2)_3-OCH_3$ | $CF_3$ | $n_D^{20}$ : 1,5040 |

Ausgangsstoffe

Die als Ausgangsstoffe verwendeten Pyrimidinderivate der Formel (II-1) bis (II-4) sind bekannt oder können in analoger Weise nach den in den unten aufgeführten Literaturstellen beschriebenen Verfahren erhalten werden:

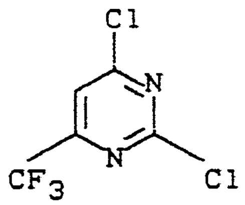

(II-1): bekannt aus:

H. Gershon et al.

J. Heterocyclic Chem. 20, 219 (1983)

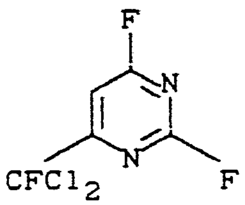

(II-2): bekannt aus:

DE-OS 3 118 699

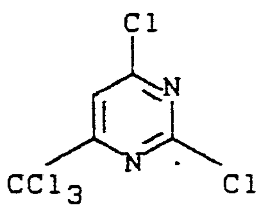

(II-3): bekannt aus:

DE-OS 3 118 699

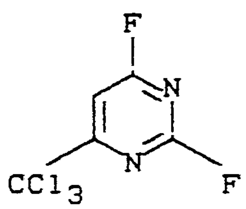

(II-4): bekannt aus:

DE-OS 3 118 699

Die noch nicht vorbeschriebenen Pyrimidinderivate (II-5) bis (II-8) können wie folgt erhalten werden:

Beispiel II-5

2,4-Difluor-6-chlordifluormethylpyrimidin

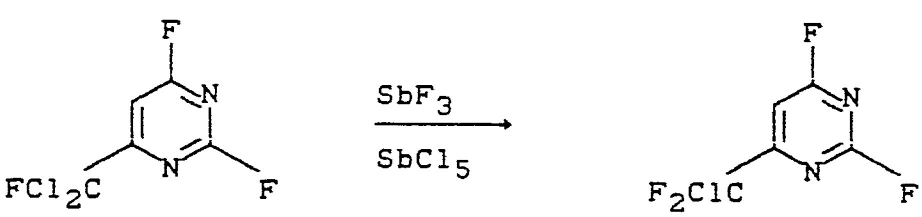


(II-2) (II-5)

In einer Rührapparatur werden 104 g 2,4-Difluor-6-dichlorfluormethylpyrimidin, 26 g Antimontrifluorid und 2,4 g Antimonpentachlorid vorgelegt und bei einer Temperatur von 154-149°C 1 Stunde bei Rückfluß gerührt. Das Reaktionsprodukt wird im Vakuum abdestilliert, mit verdünnter Salzsäure gewaschen und getrocknet. Nach Redestillation erhält man 2,4-Difluor-6-chlordifluormethyl-pyrimidin vom Siedepunkt Kp: 137-139°C, n : 1,4213

Beispiel II-6

2,4-Difluor-6-trifluormethyl-pyrimidin

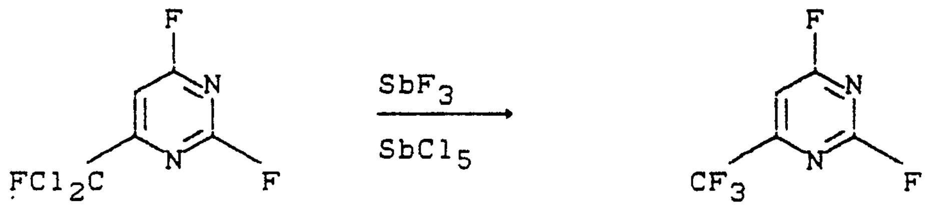


(II-2) (II-6)

In einem Rührapparatur werden 252 g 2,4-Difluor-6-dichlorfluormethyl-pyrimidin, 209 g Antimontrifluorid und 105 g Antimonpentachlorid vorgelegt und bei einer Temperatur von 130-120°C 15 Minuten bei Rückfluß gerührt. Das Reaktionsprodukt wird in leichtem Vakuum abdestilliert, mit verdünnter Salzsäure gewaschen und getrocknet. Nach Redistilation erhält man 2,4-Difluor-6-trifluormethyl-pyrimidin vom Siedepunkt Kp: 108-109°C, n : 1,3799 sowie 2,4-Difluor-6-difluorchlormethylpyrimidin (Beispiel II-5).

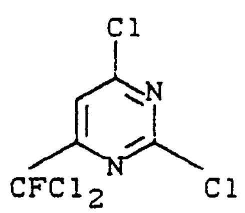

(II-7) kann in analoger Weise wie (II-1) erhalten werden

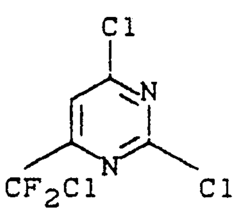

(II-8) kann in analoger Weise wie (II-1) erhalten werden

Anwendungsbeispiele

In den folgenden Anwendungsbeispielen wurden die nachstehend aufgeführten Verbindungen als Vergleichssubstanzen herangezogen:

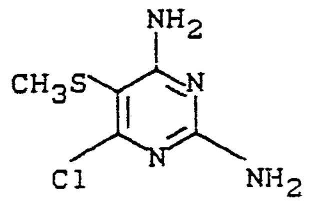

(A)

(bekannt aus EP-A-0 000 681, Beispiel 16).

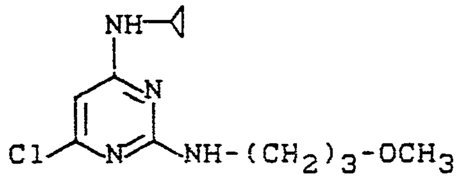

(B)

(bekannt aus DE-OS 2 630 140, Beispiel 33).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Beispielsweise zeigt die Verbindung gemäß Herstellungsbeispiel 1 in diesem Test bei gleicher Kulturpflanzenverträglichkeit in Baumwolle eine deutliche Überlegenheit in der herbiziden Wirksamkeit gegen mono-und dikotyle Unkrautpflanzen, wie Chenopodium, Datura, Stellaria, Setaria und Panicum, als die Vergleichssubstanz (A).

In diesem Test zeigt z.B. die Verbindung gemäß Herstellungsbeispiel 1 neben einer sehr guten Kulturpflanzenverträglichkeit in Baumwolle, Reis und insbesondere in Mais eine deutlich bessere herbizide Wirksamkeit gegen mono-und dikotyle Unkräuter wie Datura, Matricaria, Cynodon oder Setaria als die Vergleichssubstanz (B).

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 -15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

Die Verbindung gemäß Herstellungsbeispiel (1) zeigt z.B. in diesem Test eine bessere Kulturpflanzenverträglichkeit in Baumwolle und Mais, insbesondere in Baumwolle, und eine deutliche Überlegenheit in der herbiziden Wirksamkeit gegen mono-und dikotyle Unkräuter, wie Amaranthus, Chenopodium, Galium, Portulaca, Solanum, Echinochloa und Poa, als die Vergleichssubstanz (A).

In diesem Test zeigt z.B. die Verbindung gemäß Herstellungsbeispiel (1) eine deutlich bessere Kulturpflanzenverträglichkeit in monokotylen (wie Reis, Gerste und Weizen) und dikotylen Kulturen (wie Baumwolle) und eine deutlich bessere herbizide Wirksamkeit gegen mono-und dikotyle Unkräuter (wie Abutilon, Galium, Helianthus oder Ipomoea) als die Vergleichssubstanz (B).

**Ansprüche**

1. 2,4-Diamino-6-halogenalkylpyrimidine der Formel (I),

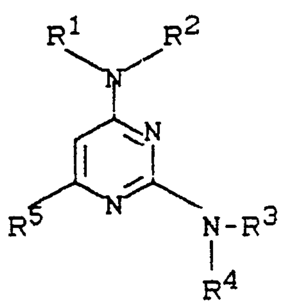

in welcher

$R^1$ und $R^3$ unabhängig voneinander für Wasserstoff oder Alkyl stehen,

$R^2$ und $R^4$ unabhängig voneinander für Wasserstoff, für gegebenenfalls durch Halogen, Cyano, Alkoxy, Alkylthio oder durch Cyclopropyl substituiertes Alkyl, für Cycloalkyl, für gegebenenfalls durch Halogen substituiertes Alkenyl oder für Alkinyl stehen und

$R^5$ für durch Halogen substituiertes Alkyl steht mit der Maßgabe, daß $R^1$, $R^2$, $R^3$ und $R^4$ nicht gleichzeitig für Wasserstoff stehen, wenn $R^5$ für Trichlormethyl steht,

mit der Maßgabe, daß $R^1$ und $R^2$ nicht gleichzeitig für Wasserstoff stehen, wenn $R^4$ für Alkyl steht, welches durch Alkoxy oder Alkylthio substituiert ist.

2. 2,4-Diamino-6-halogenalkylpyrimidinderivate der Formel (I) gemäß Anspruch 1, in welcher

$R^1$ und $R^3$ unabhängig voneinander für Wasserstoff oder für Alkyl mit 1-6 C-Atomen stehen,

$R^2$ und $R^4$ unabhängig voneinander für Wasserstoff, für gegebenenfalls durch Fluor, Chlor, Brom, Iod, Cyano oder Cyclopropyl substituiertes Alkyl mit 1-8 C-Atomen, für Alkoxyalkyl oder Alkylthioalkyl mit 1-6 C-Atomen je Alkylthio-bzw. Alkoxyteil und 2-8 C-Atomen je Alkylteil, für Cycloalkyl mit 3-5 C-Atomen, für gegebenenfalls durch Halogen substituiertes Alkenyl mit 3-6 C-Atomen oder für Alkinyl mit 3-6 C-Atomen stehen und

$R^5$ für Halogenmethyl steht,

mit der Maßgabe, daß $R^1$, $R^2$, $R^3$ und $R^4$ nicht gleichzeitig für Wasserstoff stehen, wenn $R^5$ für Trichlormethyl steht,

mit der Maßgabe, daß $R^1$ und $R^2$ nicht gleichzeitig für Wasserstoff stehen, wenn $R^4$ für Alkoxyalkyl oder Alkylthioalkyl mit 1-6 C-Atomen je Alkylthio-bzw. Alkoxyteil und 2-8 C-Atomen je Alkylteil steht.

3. 2,4-Diamino-6-halogenalkylpyrimidine der Formel (I) gemäß Anspruch 1, in welcher

$R^1$ und $R^3$ unabhängig voneinander für Wasserstoff oder für Alkyl mit 1-4 C-Atomen stehen,

$R^2$ und $R^4$ unabhängig voneinander für Wasserstoff, für gegebenenfalls durch Fluor, Chlor, Cyano oder

Cyclopropyl substituiertes Alkyl mit 1-6 C-Atomen, für Alkoxyalkyl oder Alkylthioalkyl mit 1-4 C-Atomen je Alkylthio-bzw. Alkoxyteil und 2-6 C-Atomen je Alkylteil, für Cyclopropyl oder Cyclobutyl, für gegebenenfalls durch Chlor substituiertes Alkenyl mit 3-5 C-Atomen oder für Alkinyl mit 3-5 C-Atomen stehen und

$R^5$ für durch Chlor und/oder Fluor substituiertes Methyl steht,

mit der Maßgabe, daß $R^1$, $R^2$, $R^3$ und $R^4$ nicht gleichzeitig für Wasserstoff stehen, wenn $R^5$ für Trichlormethyl steht,

mit der Maßgabe, daß $R^1$ und $R^2$ nicht gleichzeitig für Wasserstoff stehen, wenn $R^4$ für Alkoxyalkyl oder Alkylthioalkyl mit 1-4 C-Atomen je Alkylthio-bzw. Alkoxyteil und 2-6 C-Atomen je Alkylteil steht.

4. Verfahren zur Herstellung von 2,4-Diamino-6-halogenalkylpyrimidinen der Formel (I),

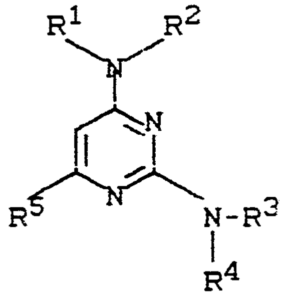

in welcher

$R^1$ und $R^3$ unabhängig voneinander für Wasserstoff oder Alkyl stehen,

$R^2$ und $R^4$ unabhängig voneinander für Wasserstoff, für gegebenenfalls durch Halogen, Cyano, Alkoxy, Alkylthio oder durch Cyclopropyl substituiertes Alkyl, für Cycloalkyl, für gegebenenfalls durch Halogen substituiertes Alkenyl oder für Alkinyl stehen und

$R^5$ für durch Halogen substituiertes Alkyl steht,

mit der Maßgabe, daß $R^1$, $R^2$, $R^3$ und $R^4$ nicht gleichzeitig für Wasserstoff stehen, wenn $R^5$ für Trichlormethyl steht,

mit der Maßgabe, das $R^1$ und $R^2$ nicht gleichzeitig für Wasserstoff stehen, wenn $R^4$ für Alkyl steht, welches durch Alkoxy oder Alkylthio substituiert ist,

dadurch gekennzeichnet, daß man Pyrimidine der Formel (II),

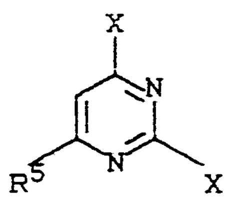

(II)

in welcher

$R^5$ die oben angegebene Bedeutung hat und

X für Chlor oder Fluor steht,

zunächst mit einem Amin der Formel (III)

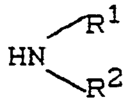

(III)

worin

$R^1$ und $R^2$ die oben angegebene Bedeutung haben,

in Gegenwart eines säurebindenden Mittels und gegebenenfalls in Gegenwart eines Verdünnungsmittels umsetzt zu einem Gemisch der isomeren Pyrimidinderivate der allgemeinen Formel (IVa) und (IVb) (1.

Stufe)

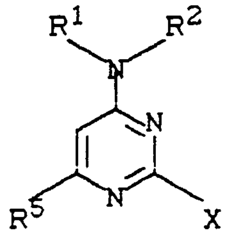

(IVa)

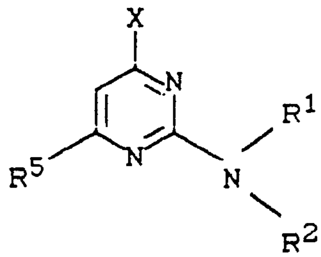

(IVb)

worin
$R^1$, $R^2$, $R^5$ und X die oben angegebene Bedeutung haben,
und anschließend gegebenenfalls nach Abtrennung der strukturisomeren Pyrimidinderivate der Formel - (IVb) in einem weiteren Reaktionsschritt mit Aminen der Formel (V),

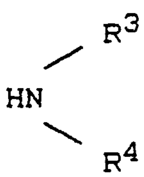

(V)

worin
$R^3$ und $R^4$ die oben angegebene Bedeutung haben,
in Gegenwart eines säurebindenden Mittels und gegebenenfalls in Gegenwart eines Verdünnungsmittels zu Pyrimidinderivaten der Formel (I) umsetzt (2. Stufe).

5. Herbizide Mittel, gekennzeichnet durch einen Gehalt an mindestens einem 2,4-Diamino-6-halogenalkylpyrimidin der Formel (I) gemäß den Ansprüchen 1 bis 4.

6. Verfahren zur Bekämpfung von Unkräutern, dadurch gekennzeichnet, daß man 2,4-Diamino-6-halogenalkylpyrimidine der Formel (I) gemäß den Ansprüchen 1 bis 4 auf die Unkräuter und/oder ihren Lebensraum einwirken läßt.

7. Verwendung von 2,4-Diamino-6-halogenalkylpyrimidinen der Formel (I) gemäß den Ansprüchen 1 bis 4 zur Bekämpfung von Unkräutern.

8. Verfahren zur Herstellung von herbiziden Mitteln, dadurch gekennzeichnet, daß man 2,4-Diamino-6-halogenalkylpyrimidine der Formel (I) gemäß den Ansprüchen 1 bis 4 mit Streckmitteln und/oder oberflächenaktiven Substanzen vermischt.

9. 4-Cyclopropylamino-2-(3-methoxypropylamino-6-trifluormethylpyrimidin der Formel